(19) **Europäisches Patentamt**

**European Patent Office**

**Office européen des brevets**

(11) **EP 1 181 951 B1**

(12) **EUROPEAN PATENT SPECIFICATION**

(45) Date of publication and mention
of the grant of the patent:
**31.03.2004 Bulletin 2004/14**

(21) Application number: **99964801.7**

(22) Date of filing: **06.12.1999**

(51) Int Cl.⁷: **A61N 1/36**

(86) International application number:
**PCT/RU1999/000471**

(87) International publication number:
**WO 2000/069516 (23.11.2000 Gazette 2000/47)**

(54) **ELECTRO-NEURO-ADAPTIVE STIMULATOR**

**ELEKTRO-NEURO-ADAPTIVER STIMULATOR**

**STIMULATEUR ELECTRO-NEURO-ADAPTATIF**

(84) Designated Contracting States:
**DE FR GB IT NL**

(30) Priority: **17.05.1999 RU 99110334**

(43) Date of publication of application:
**27.02.2002 Bulletin 2002/09**

(73) Proprietor: **Karasev, Alexandr Alexandrovich
Taganrog, 347939 (RU)**

(72) Inventor: **Karasev, Alexandr Alexandrovich
Taganrog, 347939 (RU)**

(74) Representative: **Beetz & Partner Patentanwälte
Steinsdorfstrasse 10
80538 München (DE)**

(56) References cited:
| | |
|---|---|
| **WO-A1-89/06554** | **RU-C1- 2 068 277** |
| **RU-C1- 2 091 089** | **US-A- 4 177 819** |
| **US-A- 4 431 000** | **US-A- 4 913 148** |
| **US-A- 5 800 458** | |

**Description**

TECHNICAL FIELD

The invention relates to medicine, and particularly, to electroneuroadapting stimulators, and can be used for electrostimulation procedures for medical, prophylactic and diagnostic purposes.

BACKGROUND OF THE INVENTION

Nowadays there are widely applied electrostimulators - devices that act upon the body with electric signals of various form, duration and power.

Most of such devices are of little effect because they are not supplied with the control system of the body reaction upon the electrostimulation (for example, electrostimulators, protected by the USA patents, such as №3511641, MKn. A61N1/36, in 1966; №3589370, MK΄л. A61N1/36, in 1967; №4177819, MKπ. 4 A61N1/36, in 1979; author's certificate of USSR №865300, MKπ. 4 A61N1/36, in 1981; №1034750, MKπ. 4 A61N1/36, in 1983; №1351612, MKπ. 5 A61N1/37, in 1987).

More consummate are electrostimulators that allow to determine the time of the action by the reaction of the body upon the electrostimulation. Such devices were called electroneuroadapting stimulators or bioelectric regulators of psychosomatic homeostasis, as they regulate functional condition of the body with signals, similar to nerve impulses.

Known in the art is an electrostimulator described in the author's certificate of USSR №1817335, MПK 6 A61N1/36, published in 1995, Бюл. №24, that allows to set the action dose according to the body reaction upon this action. The electrostimulator consists of a pulsed oscillator, a modulator, a power amplifier, active and passive electrodes, a differentiator, an indicator, an envelope curve former, a multiplier, and an energy stimulus controller. The disadvantage of this electrostimulator is low accuracy of determining the body adaptation to the stimulating action that leads to reduction of the therapeutic effect of the device.

Electrostimulating device, protected by the patent of Russian Federation №2091089 MПK 6 A61N1/36, published in 1997, Бюл. №27, that is unlike electrostimulator, protected by the author's certificate №1817335, additionally contains a signal conditioner, its inputs being connected to the outputs of the pulsed oscillator and the power amplifier, a time gate and a summator connected between the envelope curve former and the multiplier. It results in increasing of the speed of tracking of the body reaction upon the stimulating action, increasing of the dosage accuracy, and, consequently, the effect of treatment. The disadvantage of this electrostimulating device is low diagnostic capabilities, as diagnostics is carried out by frequency and intensity of flashes of the light-emitting diode indicator switched into the supply circuit of the power amplifier and, to a greater extent, depends upon the subjective perception of the person conducting the experiment.

A bioelectrical regulator of psychosomatic homeostasis, protected by the patent of Russian Federation №2068277, MПK 6 A61N1/36, A61H39/00, published in 1996, Бюл. №35, is supplied with higher accuracy of diagnostics (disclosing the character of pathology) and allows to prognosticate the effect of treatment.

This bioelectric regulator of psychosomatic homeostasis (electroneuroadapting stimulator) consists of a square-wave generator, connected in series; a stimulating signal forming unit; a switching-type amplifier supplied with a transformer output, connected to the active and passive electrodes; connected to the active electrode a concatenated half-wave rectifier, a measuring unit of duration and speed or the duration change of the first half-wave of forced oscillations, a unit of indication and control; and the a set up unit of parameters of stimulating signals, connected to the second input of the stimulating signal forming unit. The accuracy of diagnostics is increased due to the duration of the first half-wave of damped oscillations testifying to the character and deepness of the pathology, and the speed of the duration change testifying to the results of treatment.

The disadvantage of this bioelectric regulator of psychosomatic homeostasis, used as prior art, consists in that due to the narrow-band output of the switching-type amplifier the change of the area of action leads to the change of the level of the stimulating signal that decreases the effect of treatment and accuracy of diagnostics.

SUMMARY OF THE INVENTION

Technical achievement of application of this invention consists in creation of electroneuroadapting stimulator providing higher effect of treatment and wider diagnostic capabilities due to coordination of the level of the stimulating signal with electrophysiological parameters of the body area being acted upon.

The above mentioned technical achievement is reached by the fact that electroneuroadapting stimulator comprising the power supply; the set up unit of parameters of stimulating signal; the active and passive electrodes; and the reactive load switching-type amplifier, the active electrode being connected to one of its taps, and the passive electrode being connected to the other tap; includes a microprocessor with the set up unit of parameters of the stimulating signal being connected to its first input port; an analog-to-digital converter with its input being connected to the active electrode, and the output being connected to the second input port of the microprocessor; and the reactive load adjusting unit with its input being connected to the first output of the microprocessor, that has its second output being connected to the input of the switching-type amplifier, the reactive load of the switching-type amplifier has a component of adjustment connected to the reactive load adjusting unit; the display unit is connected to the third output of the microprocessor; and the power supply is connected to one of the taps of the reactive load of the switching-type amplifier. In accordance with the preferred embodiment of the electroneuroadapting stimulator, the reactive load of the switching-type amplifier is performed as an inductance coil having a ferromagnetic core as a component of adjustment; and the reactive load adjusting unit contains a comparator, its first input being simultaneously the input of the adjusting unit, the second input being the input of the reference voltage and the output being connected to the control winding of the micromotor that has its shaft being connected through reducer to the ferromagnetic core that is installed into the inductance coil, so that it can be displaced.

The inductance coil is performed as two-sectional, with the correlation of windings in the sections from 2:1 to 10:1, the sections being connected in series. In the reactive load of the switching-type amplifier, performed as a two-sectional inductance coil, if the point of junction of the sections is the tap for connection of the power supply, the end of the section containing less windings is the tap for connection of the switching-type amplifier, and the end of the section containing more windings is the tap for connection of the active electrode. If the point of junction of the sections is the tap for connection of the active electrode, the end of the section containing less windings is the tap for connection of the switching-type amplifier; and the end of the section containing more windings is the tap for connection of the power supply and the passive electrode. If the point of junction of the sections is the tap for connection of the switching-type amplifier and the passive electrode, the end of the section containing less windings is the tap for connection of the power supply; and the end of the section containing more windings is the tap for connection of the active electrode. If the point of junction of the sections is the tap for connection of the switching-type amplifier, the end of the section containing less windings is the tap for connection of the power supply and the passive electrode; and the end of the section containing more windings is the tap for connection of the active electrode.

The inductance coil should have high quality of 500, the inductance within 1.0±0.9 henry, and the component of adjustment should provide the change of the inductance of the coil within 0.5...1.0 of the largest extremum.

BRIEF DESCRIPTION OF THE DRAWINGS

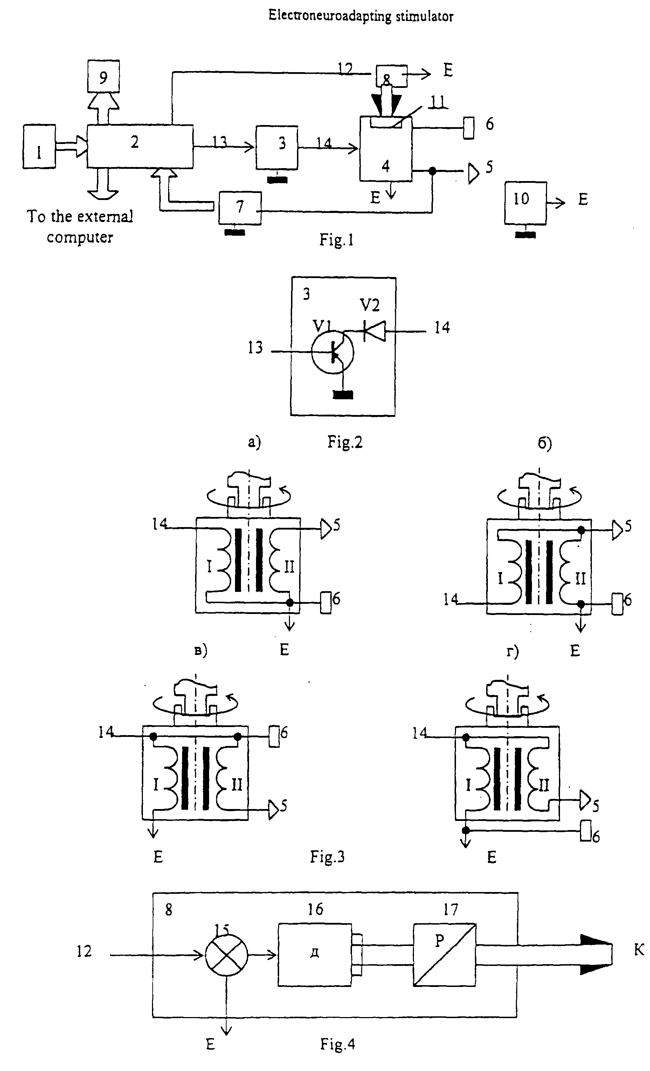

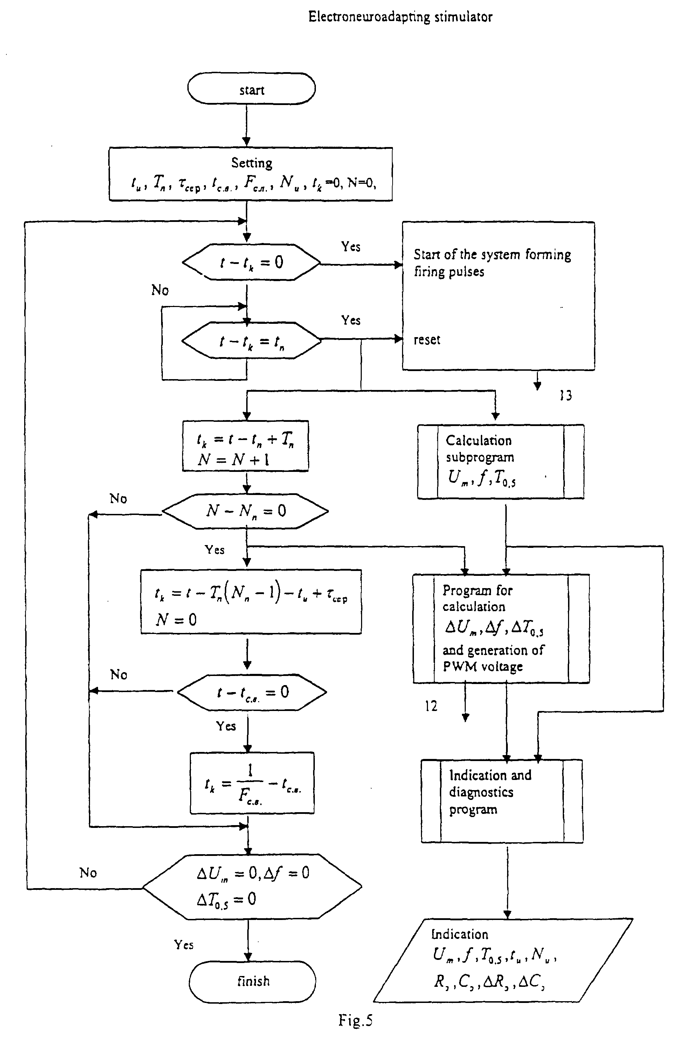

The invention is illustrated by the drawings shown in Fig.1...Fig.5.
Fig.1 shows a modular circuit of the claimed electroneuroadapting stimulator.
Fig.2 shows an electric circuit of the switching-type amplifier.
Fig.3 shows variants of connecting the two-sectional inductance coil as a reactive load of the switching-type amplifier.
Fig.4 shows a structural diagram of the reactive load adjusting unit of the switching-type amplifier.
Fig.5 shows an algorithm of operation of the microprocessor.
In Fig.1...Fig.5 there are numbered and lettered the following:

1 - set up unit of parameters of the stimulating signal;
2 - microprocessor;
3 - switching-type amplifier;
4 - reactive load of the switching-type amplifier;
5 - active electrode;
6 - passive electrode;
7 - analog-to-digital converter;
8 - reactive load adjusting unit of the switching-type amplifier;
9 - display unit;
10 - power supply;
11 - connection of the reactive load adjusting unit to the component of adjustment of the reactive load;
12 - input of the reactive load adjusting unit of the switching-type amplifier;
13 - input of the switching-type amplifier;
14 - output of connection of the switching-type amplifier to the reactive load;
15 - comparator,
16 - micromotor;
17 - reducer.
I - section of the inductance coil containing less windings;
II - section of the inductance coil containing more windings;

E - bus-bar of the power supply;

V1 - transistor (crystal triode);

V2 - crystal diode;

$t_\text{и}$ - duration of firing pulses;

$T_\text{и}$ - firing pulse repetition period;

$N_\text{и}$ - number of firing pulses in the series;

$\tau_{ccp}$ - duration of the series of firing pulses;

$t_{C.R.}$ - duration of the stimulating action;

$F_{C.D.}$ - repetition frequency of the stimulating action;

$t_K$ - control time;

t - current time;

$U_m$ - amplitude of the first half-wave of free oscillations;

f- frequency of free oscillations;

$T_{0.5}$ - time of damping of free oscillations to level of 0,5Um;

$\Delta U_m$ - change of the amplitude of the first half-wave during the duration of one series of firing pulses.

## DESCRIPTION OF THE PREFERRED EMBODIMENT

The claimed electroneuroadapting stimulator (Fig.1) consists of connected in series set up unit 1 of parameters of the stimulating signal, a microprocessor 2 and a switching-type amplifier 3 with a reactive load 4, active 5 and passive 6 electrodes being connected to it. The input of the analog-to-digital converter (ADC) 7 that converts electric potential difference between the active 5 and passive 6 electrodes into a string of codes is connected to the active electrode 5. The output of ADC 7 is connected to the second port of the input of the microprocessor 2. The reactive load 4 contains a component of adjustment that changes its reactive component within 0.5...1.0 of the largest extremum. The component of adjustment of the reactive load 4 is connected to the reactive load adjusting unit 8, having its input connected to the second output of the microprocessor 2. To the third output of the microprocessor 2 there is connected a display unit 9, meant for displaying the parameters of firing pulses and the stimulating action. The power supply 10 provides precise supply for the switching-type amplifier 3 through the reactive load 4 and reference voltage for the adjusting unit 8. In the conventional stimulator, the switching-type amplifier 3 (Fig. 2) is represented as a transistor V1, its base (input 13) being delivered firing (enabling) pulses of normed amplitude and predetermined duration from the microprocessor 2, and its collector being connected through the protecting diode V2 to one of the taps of the reactive load 4 (output 14).

In accordance with a preferred embodiment of the electroneuroadapting stimulator, the reactive load 4 is performed as a two-sectional inductance coil (Fig. 3) with the correlation of windings in the sections from 2:1 to 10:1. As a component of adjustment, the inductance coil has a ferromagnetic core 11. In this case, the reactive load adjusting unit 8 (Fig. 4) contains a comparator 15, its first input being connected to the output 12 of the microprocessor 2, the second input being connected to the bus-bar E of the power supply 10, and the output being connected to the control winding of the micromotor 16, having its shaft mechanically connected through reducer 17 to the ferromagnetic core 11.

Variants of connection of the taps of the reactive load 4 are shown in Fig. 3a...Fig. 3r.

Fig. 3a shows the point of junction of the sections I and II being connected to the passive electrode 6 and the bus-bar E of the power supply 10, the section I containing less windings being connected to the output 14 of the switching-type amplifier 3, and the section 11 containing more windings being connected to the active electrode 5. Fig. 36 shows the point of junction of the sections I and II being connected to the active electrode 5, the section I being connected to the output 14 of the switching-type amplifier 3, and the section II being connected to the passive electrode 6 and the bus-bar E of the power supply 10. Fig. 3b shows the point of junction of the sections I and II being connected to the output 14 of the switching-type amplifier 3 and the passive electrode 6, the section I being connected to the bus-bar E of the power supply 10, the section II being connected to the active electrode 5. Fig. 3r shows the point of junction of the sections I and II being connected to the output 14 of the switching-type amplifier 3, the section I being connected to the bus-bar E of the power supply 10 and the passive electrode 6, the section II being connected to the active electrode 5.

The principle of operation of the electroneuroadapting stimulator is based upon the fact that stimulating signals, that act upon the tissue structure under the electrode are electric oscillations in an oscillatory circuit formed by connected in series reactive load and capacitive and reactive components of impedance of the tissue structure under the electrode, appearing together with stopping of action of firing pulse on the input of the switching type amplifier 3 after it has been disconnected. Parameters of these oscillations (amplitude, frequency, damping time) to 90...95% depend only upon electrophysiological condition of the tissue structure under the electrode.

It depends on the constancy of level of oscillations energy, stability of the parameters of the reactive load and elimination of influence of impedance of contact "electrode - tissue under the electrode". Stability of the parameters of the reactive load is achieved by a high-quality inductance coil (with high quality over 500). Constancy of the oscillations energy is achieved due to being equal to the energy accumulated in the reactive load with the switching-type amplifier 3 being switched on and depends only upon the duration of firing pulse. To exclude any influence upon the parameters of the electric oscillations of impedance of contact "electrode - tissue under the electrode", it is enough to use the electrodes having the contact area over 5 mm$^2$ and choose resonance frequency of the oscillatory circuit over 100 kHz at the expense of the parameters of the reactive load. Under such conditions, the value of the capacitive component of impedance of contact "electrode - tissue under the electrode" is two orders less than the value of the capacitive component of impedance of the tissue structures under the electrode. Thus, with high stability of firing pulses, characteristics of the switching-typc amplifier 3, the parameters of the reactive load 4, that can be easily realized under modern technical conditions, and, provided that the contact area is over 5 mm$^2$ and the resonance frequency of the oscillatory circuit is over 100 kHz, the parameters of free oscillations in the oscillatory circuit will reflect biochemical processes and physical condition of the tissue structures under the electrode with high accuracy, as the active component of impedance of these structures characterizes blood filling and admittance of the intertissular medium and capacitive component characterizes cellular and intercellular polarization. Disbalance of the biochemical processes under the influence of the stimulating signal will cause the changing of both active and reactive component of the impedance of the tissue structures under the electrode. In its turn, this changing causes changing of the parameters of free oscillations in the oscillatory circuit formed by series connection of the inductance ot the reactive load 3 and the capacitive component of impedance of the tissue structures under the electrode. Measuring the parameters of these oscillations allows to judge about the body reaction upon the stimulating action. When the balance of the biochemical processes is achieved, the stimulating action stops its influence upon the value of impedance of the tissue structures under the electrode and it should be stopped, as overdose of the stimulating action may lead to negative result. Therapeutic effect of applying the electroneuroadapting stimulator of psychosomatic homeostasis in many respects depends upon the accuracy of determining the moment when the balance of the biochemical processes is achieved, i.e. when the impedance of the tissue structures under the electrode stops changing under the influence of the stimulating signal.

Operation of the claimed electroneuroadapting stimulator of psychosomatic homeostasis is described taking into account the algorithm of operation of the microprocessor 2 shown in Fig. 5.

Switching on the electroneuroadapting stimulator is carried out with the set up unit 1 of parameters of the stimulating signal that is performed as a push-button control panel and the parameters of firing pulses - duration of firing pulses $t_и$, firing pulse repetition period $T_и$, number of firing pulses in scries $N_H$, duration of the series of firing pulses $\tau_{ccp}$, duration of the stimulating action $t_{C.R.,}$ and repetition frequency of the stimulating action $F_{C.R.}$ are recorded into on-line storage of the microprocessor 2. After the parameters of firing pulses have been recorded into the on-line storage of the microprocessor 2, the timer is activated, control time and current number of pulse are set to zero and there starts counting of the current time and comparing it with the control time. If the current time is equal to the control one ($t-t_K=0$), firing pulse-shaping circuit is started and it is reset when the difference between the current and control time is equal to $t_H$. Pulses with normed amplitude are delivered from the output of the firing pulse-shaping circuit to the input 13 of the switching-type amplifier 3 and switch it on. During action of firing pulse, there is conducted electric current through the inductance coil of the reactive load 4 and inside the inductance coil there is accumulated electromagnetic energy that can be determined by the following expression:

$$\Theta = \frac{L}{2}\left[\frac{E}{r_0+r_L+r_{кл}}\left(1-e^{-\frac{r_0+r_L+r_{кл}}{L}\cdot t_и}\right)\right]^2$$

L - is value of the inductance of the reactive load 4,
E - is voltage of the power supply 10,
$r_0$ - is internal resistance of the power supply 10,
$r_L$ - is ohmic resistance of the reactive load 4,
$r_{кл}$ - is resistance of the open switching-type amplifier 3,
$t_и$ - is duration of the pulse.

After the action of firing pulse has been stopped, the switching-type amplifier 3 is switched off and in the oscillatory circuit, formed by series connection of the inductance of the reactive load 4, equivalent capacitance C, and equivalent ohmic resistance R, of the tissue structures under the electrode, there appear electric oscillations that can be described by the following expression:

$$U(t) = U_m \cdot e^{-at} \cdot \cos(2\pi f t) =$$

$$= \sqrt{\frac{L}{C_\ni} \cdot \frac{E}{r_0 + r_L + r_{\kappa\lambda}}\left(1 - e^{-\frac{r_0 + r_L + r_{\kappa\lambda}}{L} \cdot t_u}\right)} \cdot e^{-ut} \cdot \cos\left(2\pi\sqrt{\frac{L}{C_\ni} - \frac{R_\ni}{4L^2}} \cdot t\right)$$

$U_m$ - is amplitude of the first half-wave of oscillations;
$\alpha$ - is speed of oscillation damping;
$f$ - is frequency of oscillations;
L - is inductance of the reactive load 4;
$C_\ni$ - is equivalent capacitance of the tissue structures under the electrode;
E - is voltage of the power supply 10;
$r_0$ - is internal resistance of the power supply 10;
$r_L$ - is ohmic resistance of the reactive load 4;
$r_{\kappa\lambda}$ - is resistance of the switching-type amplifier 3 being switched on;
$t_H$ - is duration of the firing pulse;
$R_O$ - is equivalent ohmic resistance of the tissue structures under the electrode.

From the output 7 of ADC, voltage codes between the active 5 and passive 6 electrodes are read with the frequency that is 10 - 20 times higher than the oscillations frequency and are delivered to the microprocessor 2. In the microprocessor 2, supplied with special subprogram, there are determined $U_m$ - amplitude of the first half-wave of oscillations, $f$ - frequency of oscillations and $T_{0.5}$ - time of damping of amplitude of oscillations to level 0.5. Then the control time is set equal to the time of start of the next firing pulse ($t_K \Delta t - t_u + T_u$ ) and 1 is added to the current number of the pulse ($N \Delta N + 1$); after that the new number of the pulse is compared with the set value of pulses in N series and, if they are not equal, another firing pulse is formed and new values $U_m$, $f$, and $T_{0.5}$ are determined; if they are equal, the control time is set equal to the time of start of new series of firing pulses ( $t_u \Delta t - T_u(N-1) - t_K + \tau_{ccp}$), the current number of the pulse is set equal to zero ($N \Delta 0$) and subprogram of trimming of the inductance coil of the reactive load 4 is started. According to this program, change $U_m$ during the duration of series of firing pulses is determined and, in accordance with the value of this change, PWM voltage delivered to the input 8 of the reactive load adjusting unit is corrected, and with the help of the component of adjustment - the ferromagnetic core 11 the inductance of the inductance coil is being changed until the value $U_m$ is not equal to the initial value. This allows to increase the accuracy of determining the body reaction upon the stimulating action, as when the equivalent capacitance of the impedance of the tissue structures under the electrode is being changed, wave resistance of the oscillatory circuit is maintained constant.
The sign of no reaction of the body upon the stimulating action and termination of the stimulation is determined by constancy of at least one of parameters of electric oscillations ($U_m$, $f$, and $T_{0.5}$) during the next series of firing pulses, i.e. when the following condition is executed:

$\Delta U_m = U_{m1} - U_{mNH} = 0$, or $\Delta f = \Delta f_1 - \Delta f_{NH} = 0$, or $\Delta T_{0.5} = T^1_{0.5} - T^N_{0.5} = 0$;

$\Delta U_m$ - is change of the amplitude of the first half-wave of electric oscillations during the duration of series of firing pulses;
$U_{m1}$ - is the amplitude of the first half-wave of electric oscillations from the first pulse in the series;
$U_{mNH}$ - is the amplitude of the first half-wave of electric oscillations from the last pulse in the series;
$\Delta f$ - is the change of the frequency during the series of firing pulses;
$f_1$ - is the frequency of electric oscillations after the first pulse in the series;
$f_{NM}$ - is the frequency of electric oscillations after the last pulse in the series;
$\Delta T_{0.5}$ - is the change of time of damping of amplitude of free oscillations to level 0,5 during the duration of the series of firing pulses;
$T^1_{0.5}$ - is the time of damping of amplitude of oscillations to level 0,5 after the first pulse in the series;
$T^N_{0.5}$ - is the time of damping of amplitude of oscillations to level 0,5 after the last pulse in the series.

Constancy of one of the parameters of electric oscillations during the action of the stimulating signals ($N_u$ signals with pulse repetition period $\tau_u$) testifies to the electrophysiological condition of the tissue structures under the electrode being unchanged or changed within the sensitivity of the device; the further action may convert it from tonic into stress one and eliminate therapeutic effect.

Execution of one of those conditions will lead to switching off of the device.

In accordance with the subprogram of indication and diagnostics, there are generated signals for reflecting the characteristics allowing to judge objectively about the electrophysiological condition of the tissue structures under the electrode before the stimulating action and after that. Such characteristics are power level of the stimulating action ($t_\text{и}$ and $N_\text{и}$ are reflected), values of the active and reactive components of impedance of the tissue under the electrode at the beginning and at the end of the action (there reflected $R_\text{э}$, $C_\text{э}$, $\Delta R_\text{э}$ and $\Delta C_\text{э}$ - the final values of the active and reactive components of impedance and their change during the time of the action).

The effect of therapeutic action is increased due to the increase of accuracy of dosage of the stimulating action. Diagnostic capabilities are enlarged due to objectivizing of the results of the action.

The claimed electroneuroadapting stimulator can be easily made of the components available in industry. The microprocessor 2 ADC 7 can be made of programmable logic devices FLEX 10k, the switching-type amplifier can be made of KT815 transistor. An inductance coil with the cup core Б-18, Б-22 can be used as the inductance coil.

**Claims**

1.  Electroneuroadapting stimulator comprising: a power supply; a set up unit of parameters of stimulating signal; active and passive electrodes, and a reactive load switching-type amplifier, the active electrode being connected to one of its taps, and the passive electrode being connected to the other tap; **characterized in that**, it includes as its components a microprocessor with the set up unit of parameters of stimulating signal being connected to its first input port; an analog-to-digital converter with its input being connected to the active electrode, and the output being connected to the second input port of the microprocessor, a reactive load adjusting unit with its input being connected to the first output of the microprocessor, that has its second output connected to the input of the switching-type amplifier, the reactive load of the switching-type amplifier containing a component of adjustment connected to the reactive load adjusting unit; a display unit being connected to the third output of the microprocessor, and the power supply being connected to one of the taps of the reactive load of the switching-type amplifier.

2.  Electroneuroadapting stimulator as claimed in Claim 1, **characterized in that**, the reactive load of the switching-type amplifier is performed as an inductance coil having a ferromagnetic core as a component of adjustment; and the reactive load adjusting unit containing a comparator, its first input being simultaneously the input of the adjusting unit, the second input being the input of the reference voltage and the output being connected to control winding of a micromotor that has its shaft being connected through reducer to the ferromagnetic core that is installed into the inductance coil, so that it can be displaced.

3.  Electroneuroadapting stimulator as claimed in Claim 2, **characterized in that**, the inductance coil is performed as two-sectional, with the correlation of windings in the sections from 2:1 to 10:1, the sections being connected in series.

4.  Electroneuroadapting stimulator as claimed in Claim 3, **characterized in that**, in the reactive load of the switching-type amplifier, performed as a two-sectional inductance coil, the point of junction of the sections is the tap for connection of the power supply and the passive electrode, the end of the section containing less windings is the tap for connection of the switching-type amplifier, and the end of the section containing more windings is the tap for connection of the active electrode.

5.  Electroneuroadapting stimulator as claimed in Claim 3, **characterized in that**, in the reactive load of the switching-type amplifier, performed as a two-sectional inductance coil, the point of junction of the sections is the tap for connection of the active electrode, the end of the section containing less windings is the tap for connection of the switching-type amplifier, and the end of the section containing more windings is the tap for connection of the power supply and the passive electrode.

6.  Electroneuroadapting stimulator as claimed in Claim 3, **characterized in that**, in the reactive load of the switching-type amplifier, performed as a two-sectional inductance coil, the point of junction of the sections is the tap for connection of the switching-type amplifier and the passive electrode, the end of the section containing less windings is the tap for connection of the power supply, and the end of the section containing more windings is the tap for connection of the active electrode.

7.  Electroneuroadapting stimulator as claimed in one of the Claims 2...6, **characterized in that**, the inductance coil has high quality over 500, inductance within 1.0±0.9 henry, and the component of adjustment provides the change of the inductance of the coil within 0.5...1.0 of the largest extremum.

**Patentansprüche**

1. Elektroneuroadaptiver Stimulator, der aufweist: eine Stromquelle, eine Parametereinstelleinheit für das stimulierende Signal, aktive und passive Elektroden, und einen Blindlast-Schaltverstärker, wobei die aktive Elektrode an einen seiner Abgriffe und die passive Elektrode an den anderen Abgriff angeschlossen ist, **dadurch gekennzeichnet, daß** er als Bauteil einen Mikroprozessor enthält, an dessen ersten Eingangsanschluss die Parametereinstelleinheit für das stimulierende Signal angeschlossen ist; einen Analog-Digital-Wandler, dessen Eingang an die aktive Elektrode und dessen Ausgang an den zweiten Eingangsanschluss des Mikroprozessors angeschlossen ist; eine Blindlast-Einstelleinheit, deren Eingang mit dem ersten Ausgang des Mikroprozessors verbunden ist, dessen zweiter Ausgang mit dem Eingang des Schaltverstärkers verbunden ist, wobei die Blindlast des Schaltverstärkers ein Einstellbauteil enthält, das mit der Blindlast-Einstelleinheit verbunden ist, wobei eine Anzeigeeinheit mit dem dritten Ausgang des Mikroprozessors verbunden ist, und wobei die Stromquelle mit einem der Abgriffe der Blindlast des Schaltverstärkers verbunden ist.

2. Elektroneuroadaptiver Stimulator nach Anspruch 1, **dadurch gekennzeichnet, daß** die Blindlast des Schaltverstärkers als eine Drosselspule mit einem ferromagnetischen Kern als Einstellbauteil ausgeführt ist; und die Blindlast-Einstelleinheit einen Komparator enthält, dessen erster Eingang gleichzeitig der Eingang der Einstelleinheit ist, dessen zweiter Eingang der Eingang für die Referenzspannung und der Ausgang mit der Steuerwicklung eines Mikromotors verbunden ist, dessen Welle über ein Untersetzungsgetriebe mit dem in die Drosselspule eingebauten ferromagnetischen Kern verbunden ist, so daß sie verschoben werden kann.

3. Elektroneuroadaptiver Stimulator nach Anspruch 2, **dadurch gekennzeichnet, daß** die Drosselspule zweiteilig mit einem Wicklungsverhältnis in den Teilen von 2 : 1 bis 10 : 1 ausgeführt ist, wobei die Teile in Reihe geschaltet sind

4. Elektroneuroadaptiver Stimulator nach Anspruch 3, **dadurch gekennzeichnet, daß** in der als zweiteilige Drosselspule ausgeführten Blindlast des Schaltverstärkers der Verbindungspunkt der Teile der Abgriff für den Anschluß der Stromquelle und der passiven Elektrode ist, das Ende des Teils mit kleinerer Wicklungszahl der Abgriff für den Anschluß des Schaltverstärkers ist, und das Ende des Teils mit größerer Wicklungszahl der Abgriff für den Anschluß der aktiven Elektrode ist.

5. Elektroneuroadaptiver Stimulator nach Anspruch 3, **dadurch gekennzeichnet, daß** in der als zweiteilige Drosselspule ausgeführten Blindlast des Schaltverstärkers der Verbindungspunkt der Teile der Abgriff für den Anschluß der aktiven Elektrode ist, das Ende des Teils mit kleinerer Wicklungszahl der Abgriff für den Anschluß des Schaltverstärkers ist, und das Ende des Teils mit größerer Wicklungszahl der Abgriff für den Anschluß der Stromquelle und der passiven Elektrode ist.

6. Elektroneuroadaptiver Stimulator nach Anspruch 3, **dadurch gekennzeichnet, daß** in der als zweiteilige Drosselspule ausgeführten Blindlast des Schaltverstärkers der Verbindungspunkt der Teile der Abgriff für den Anschluß des Schaltverstärkers und der passiven Elektrode ist, das Ende des Teils mit kleinerer Wicklungszahl der Abgriff für den Anschluß der Stromquelle, und das Ende des Teils mit größerer Wicklungszahl der Abgriff für den Anschluß der aktiven Elektrode ist.

7. Elektroneuroadaptiver Stimulator nach einem der Anspruch 2 bis 6, **dadurch gekennzeichnet, daß** die Drosselspule eine hohe Qualität von mehr als 500 hat, eine Induktivität innerhalb von 1,0±0,9 H, und das Einstellbauteil die Induktivitätsänderung der Spule im äußersten Grenzwertbereich innerhalb von 0,5...1,0 liefert.

**Revendications**

1. Stimulateur électro-neuro-adaptif comprenant: une source d'énergie; une unité de réglage des paramètres du signal stimulant, des électrodes active et passive, et un amplificateur à charge réactive du type à commutation, l'électrode active étant connectée à une de ses prises, et l'électrode passive étant connectée à l'autre prise, **caractérisé par le fait qu'**il comporte en tant que composants un microprocesseur, l'unité de réglage des paramètres du signal stimulant étant connectée à sa première borne d'entrée; un convertisseur analogique-numérique ayant son entrée connectée à l'électrode active et sa sortie connectée à la seconde borne d'entrée du microprocesseur; une unité d'ajustage de la charge réactive ayant son entrée connectée à la première sortie du microprocesseur, dont la seconde sortie est connectée à l'entrée de l'amplificateur du type à commutation; la charge réactive de l'amplificateur du type à commutation comprenant un composant d'ajustage connecté à l'unité d'ajustage de la charge réactive; une unité d'affichage étant connectée à la troisième sortie du microprocesseur, et la source d'énergie étant connectée à une des prises de la charge réactive de l'amplificateur du type à commutation.

2. Stimulateur électro-neuro-adaptif selon la revendication 1, **caractérisé par le fait que** la charge réactive de l'amplificateur du type à commutation est réalisée sous forme d'une bobine à inductance ayant un noyau ferromagnétique comme élément d'ajustage; et l'unité de réglage de la charge réactive comporte un comparateur dont la première entrée est simultanément l'entrée de l'unité d'ajustage, la seconde entrée étant l'entrée de la tension de référence et la sortie étant connectée à l'enroulement de commande d'un micromoteur dont l'arbre est connecté par l'intermédiaire d'un réducteur au noyau ferromagnétique installé dans la bobine à inductance tel qu'il peut être déplacé.

3. Stimulateur électro-neuro-adaptif selon la revendication 2, **caractérisé par le fait que** la bobine à inductance est conformée en deux sections, la corrélation des enroulements dans les sections étant de 2:1 à 10:1, les sections étant connectées en série.

4. Stimulateur électro-neuro-adaptif selon la revendication 3, **caractérisé par le fait que**, dans la charge réactive de l'amplificateur du type à commutation, conformée sous forme d'une bobine à inductance en deux sections, le point de jonction des sections est la prise de connexion pour la source d'énergie et l'électrode passive, l'extrémité de la section contenant moins d'enroulements étant la prise pour la connexion de l'amplificateur du type à commutation, et l'extrémité de la section comportant plus d'enroulements étant la prise pour la connexion de l'électrode active.

5. Stimulateur électro-neuro-adaptif selon la revendication 3, **caractérisé par le fait que**, dans la charge réactive de l'amplificateur du type à commutation, conformée sous forme d'une bobine à inductance en deux sections, le point de jonction des sections est la prise pour la connexion de l'électrode active, l'extrémité de la section contenant moins d'enroulements étant la prise pour la connexion de l'amplificateur du type à commutation, et l'extrémité de la section contenant plus d'enroulements étant la prise pour la connexion de la source d'énergie et l'électrode passive.

6. Stimulateur électro-neuro-adaptif selon la revendication 3, **caractérisé par le fait que**, dans la charge réactive de l'amplificateur du type à commutation, conformée sous forme d'une bobine à inductance en deux sections, le point de jonction des sections est la prise pour la connexion de l'amplificateur du type à commutation et de l'électrode passive, l'extrémité de la section contenant moins d'enroulements étant une prise pour la connexion de la source d'énergie, et l'extrémité de la section contenant plus d'enroulements étant une prise pour la connexion de l'électrode active.

7. Stimulateur électro-neuro-adaptif selon l'une des revendications 2 à 6, **caractérisé par le fait que** la bobine à inductance a une qualité élevée de plus que 500, une inductance dans les limites de $1,0 \pm 0,9$ henry, et le composant d'ajustage fournit le changement de l'inductance de la bobine dans les limites de 0,5 .... 1,0 de la valeur maximale.

Electroneuroadapting stimulator

Fig.1

To the external
computer

Fig.2

a)    б)

в)    г)

Fig.3

Fig.4

Electroneuroadapting stimulator

start

Setting
$t_u$, $T_n$, $\tau_{ccp}$, $t_{c.s.}$, $F_{c.n.}$, $N_u$, $t_k = 0$, N=0,

$t - t_k = 0$ — Yes → Start of the system forming firing pulses

No

$t - t_k = t_n$ — Yes → reset

13

$t_k = t - t_n + T_n$
$N = N + 1$

Calculation subprogram
$U_m, f, T_{0.5}$

$N - N_n = 0$ — No

Yes

$t_k = t - T_n(N_n - 1) - t_u + \tau_{ccp}$
$N = 0$

Program for calculation
$\Delta U_m, \Delta f, \Delta T_{0.5}$
and generation of PWM voltage

12

$t - t_{c.s.} = 0$ — No

Yes

$t_k = \dfrac{1}{F_{c.s.}} - t_{c.s.}$

Indication and diagnostics program

$\Delta U_m = 0, \Delta f = 0$
$\Delta T_{0.5} = 0$ — No

Yes

finish

Indication
$U_m, f, T_{0.5}, t_u, N_u,$
$R_,, C_,, \Delta R_,, \Delta C_,$

Fig.5